# EUROPEAN PATENT APPLICATION

(11) **EP 2 740 509 A1**
(43) Date of publication of application: **11.06.2014**
(21) Application number: 13163243.2
(22) Date of filing: 11.04.2013
(51) Int. Cl.: A61M 16/06, B29C 33/42

(54) **Anti-slip face mask**

(30) Priority: 10.12.2012 TW 101223869 U
(71) Applicant: Besmed Health Business Corp., New Taipei City 248 (TW)
(72) Inventor: Shyong, Bill, Cambridge, CB1 1AH (GB)
(74) Representative: Horak, Michael

(57) **Abstract**

The present invention relates to the improvement in a face mask (1) for medical use, respiration anesthesia or first aid use. The face mask (1) is structured from a main body casing (10) made of hard plastic, with a tubular shaped air inlet (11) protruding from the surface of the casing (1); and a mouth-shaped adhering piece (12) made of soft elastic plastic. The adhering piece (12) is formed on the peripheral edge of the opening of the casing (10), and is used to adhere to the user's face. Anti-slip portions (13a, 13b) that are formed as an integral body with the soft adhering piece (12) are provided on at least one portion of the surface of the casing (10).

Presence of the anti-slip portions (13a, 13b) is used to enable health care personnel to easily hold the face mask (1) and prevent the face mask (1) from slipping when fitting the face mask (1) to a patient or when removing the face mask (1) to examine the patient or for cleaning and sterilizing.

The anti-slip portions (13a, 13b) may be formed as protruding strips (131, 133), sheet forms (134), protruding points (132) or a mixed configuration thereof. Because the anti-slip portions (13a, 13b) are formed as an integral body extending from the adhering piece (12), thus, very little material is used, and increase in cost is limited, but the improvements made make the face mask more convenience to use.

## Description

### BACKGROUND OF THE INVENTION

### a) Field of the Invention

The present invention relates to a face mask for medical use, respiration anesthesia or first aid use, and more particularly to an anti-slip face mask in which the surface of a hard face mask casing is provided with anti-slip portions formed as an integral body with a soft elastic mouth-shaped piece joined to the peripheral edge of the opening of the casing.

### b) Description of the Prior Art

In the past, a medical use face mask, used to cover the nose and mouth of the face of a patient for delivering oxygen or anesthetic drugs, was usually structured from a casing made from hard plastic such as polycarbonate, and the like, and formed as an inverted bowl shape; and a mouth-shaped adhering piece (hereinafter, at times, referred to as the mouth-shaped piece) usually molded from soft elastic plastic, such as silicone, synthetic rubber or resin plastic, and formed on the peripheral edge of the casing portion for use to enable the face mask to tightly adhere to the patient's face. A short tubular air inlet protrudes from the casing and can be connected to a gas tube (not shown in the drawings). The air inlet is used to deliver oxygen or anesthetic gases into the interior of the face mask for patients to breathe in. And the soft, elastic characteristics of the adhering piece is used to fit onto the face to prevent gas leakage.

According to such face masks of the prior art, because of the smooth bowl-shaped surface of the hard casing, the face mask is slippy and difficult to hold. As a result, when health care personnel are fitting the face mask to a patient or temporarily removing the face mask to examine the patient or for cleaning and sterilizing, the face mask easily slips and drops, thus causing inconvenience in use.

Although the face mask disclosed in Taiwan patent No. M270826 is a medical use face mask, however, it mainly provides a gas discharge device for use by the face mask. The surface of the main body of the face mask casing is still smooth and slippery, and provides no anti-slip devices.

In addition, in the medical use face mask disclosed in Taiwan patent 468477, the cover and the adhering portion used to adhere to the face of the patient forms a structure able to be dismantled from and assembled to the opening edge of the cover, and the adhering portion forms a tubular member. After inwardly turning over and close integration, a circular tube may be formed which can be filled with air. This structure has no relation to an anti-slip device.

### SUMMARY OF THE INVENTION

The objective of the present invention is to eliminate the aforementioned existing problems in the medical use face masks of the prior art, and to provide a medical use face mask having anti-slip portions on portions of the surface of a hard plastic casing, the anti-slip portions being made from the same material as a soft mouth-shaped piece (that is, an adhering piece) connected to the peripheral edge of the opening of the casing.

The anti-slip portions are preferably provided at two side positions on the surface of the casing and positioned at the front and rear of an air inlet tube, or to the left and right of the air inlet tube. Moreover, the anti-slip portions are a plurality of thin protruding strips which extend along the circumferential direction of the casing, or are a plurality of fine protruding points or sheet form bodies or other appropriate structural forms,

It is preferred that the anti-slip portions connectedly extend from the soft mouth-shaped piece and are formed as an integral body with the mouth-shaped piece.

To enable a further understanding of said objectives and the technological methods of the invention herein, a brief description of the drawings is provided below followed by a detailed description of the preferred embodiments.

### BRIEF DESCRIPTION OF THE DRAWINGS

FIG. 1 shows a perspective view of an embodiment of the present invention.
FIG. 2 shows a top view of the embodiment of the present invention.
FIG. 3 shows a cutaway view along line A-A of FIG. 2.
FIG. 4 shows a bottom view along line A-A of FIG. 2.
FIG. 5 shows a schematic view of examples of different forms of anti-slip portions.
FIG: 6 shows a perspective view of another embodiment of the present invention.
FIG. 7 shows a top view of the other embodiment of the present invention.

### DETAILED DESCRIPTION OF THE PREFERRED EMBODIMENTS

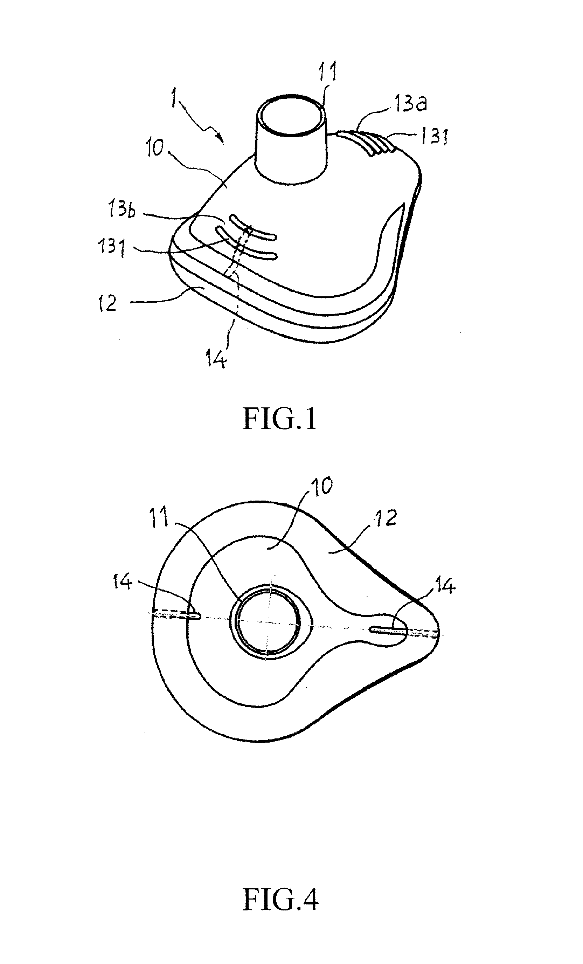

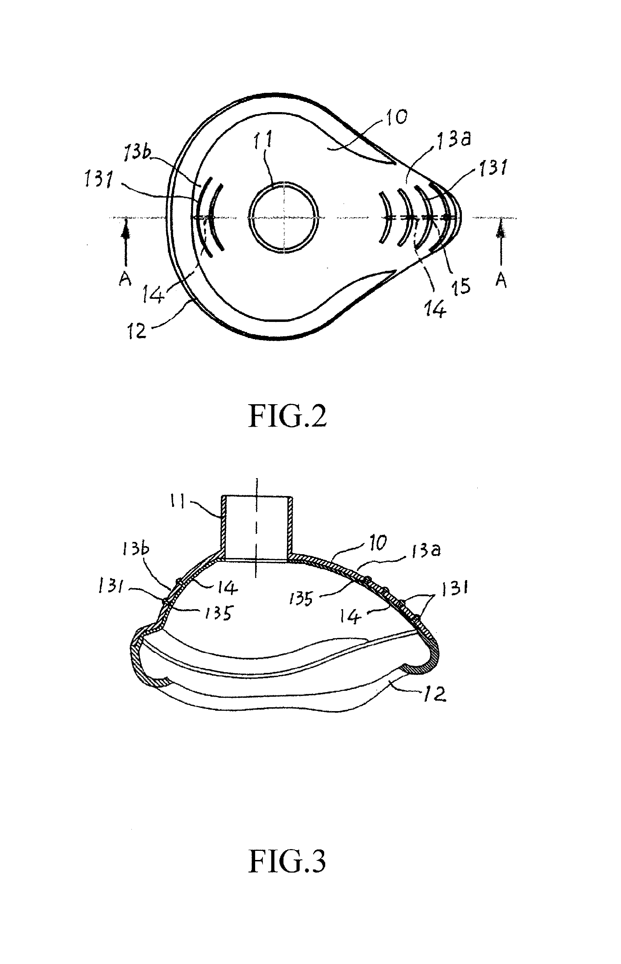

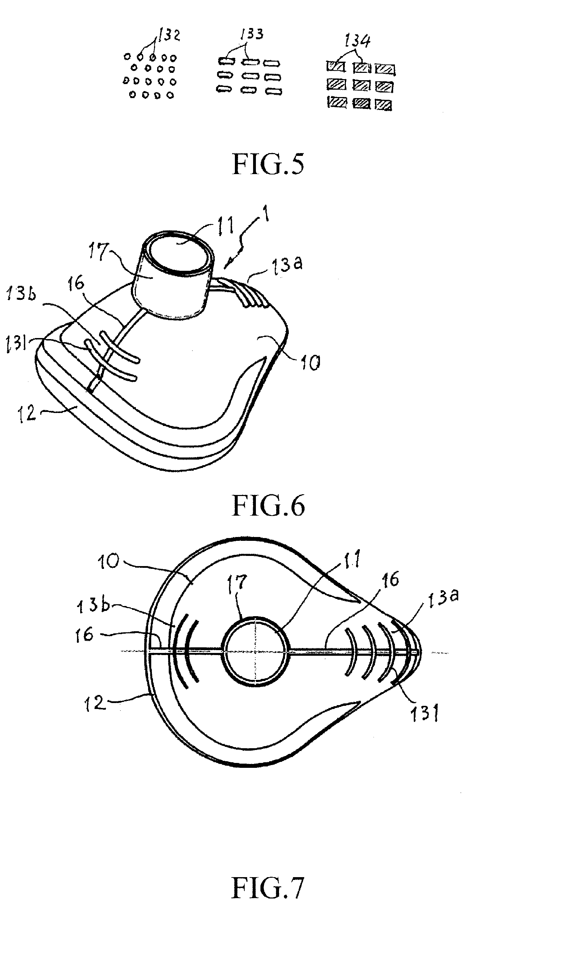

Referring to FIGS. 1 to 4, which show a face mask 1 of the the present invention structured from a casing 10 somewhat similar in shape to a funnel and formed using hard plastic, a tubular air inlet 11 that protrudes from the top portion slightly to the center of the surface of the casing 10, and a mouth-shaped piece 12 made from soft elastic plastic and formed as an integral body with the opening peripheral edge of the casing 10. The air inlet 11 can be connected to a gas delivery tube (not shown in the drawings) to allow delivery of gas such as oxygen or gaseous drugs to enter the interior of the face mask 1, and the mouth-shaped piece 12 can be adhered to the face of a patient to prevent gas leakage. Anti-slip portions 13a, 13b are provided on the surface of the casing 10 and positioned at the peripheral portions of the air inlet 11. In this embodiment. the anti-slip portions 13a, 13b are respectively structured from a plurality of thin protruding strips 131 positioned at the front and rear portions of the air inlet 11 and extending along the shaped circumferential direction of the casing 10. However, the anti-slip portions 13a, 13b are not limited to such protruding strips, and any form can be used as long as they can achieve an anti-slip elect. For example, as shown in FIG. 5, the formation of a plurality of protruding points 132 or disjoined protruding strips 133 or long or short sheet-like bodies 134 provided with rough surfaces, or other shaped structural forms are equally appropriate. In addition, the anti-slip portions 13a, 13b are not limited to being positioned at the front and rear portions of the casing 10, and can also be positioned on the left and right positions or other appropriate positions.

In order to form the anti-slip portions 13a, 13b from the same material as that of the mouth-shaped piece 12, the molding method used is to provide a feed channel 14 inside each of the two ends of the casing 10 corresponding to the proposed anti-slip portions 13a, 13b, or one feed channel that goes from one side to the other side of the casing 10 bypassing the air inlet 11. The feed channels 14 are respectively provided with a feed outlet hole 15 which penetrates the casing 10 and corresponds to each of the positions of the anti-slip protruding strips 131. The mold is first provided with feed grooves which are used to form the protruding strips 131. Accordingly. when the molten material of the mouth-shaped piece 12 is within the mold and taking shape, a portion of the material will pass through the feed channels 14 and the feed outlet hole 15 and flow to the surface of the casing 10 to form the anti-slip portions 13a, 13b. Hence, the mouth-shaped piece 12 and the anti-slip portions 13a, 13b are formed as an integral body.

FIG. 6 and FIG. 7 show another embodiment of the face mask of the present invention. In this embodiment, the structure of the face mask 10 is basically the same as the foregoing embodiment, the only difference being in the different method used to form the anti-slip portions 13a, 13b compared to the aforementioned method. In this embodiment, the molten material of the mouth-shaped piece 12 passes through a feed channel 16 and branches off into two sides to form the anti-slip portions 13a, 13b. Accordingly, apart from the transverse protruding strips 131 of the anti-slip portions 13a, 13b, the central portion of the casing 10 includes a protruding strip 16 formed from the feed channel.

In order to avoid a deficiency in the molten material or poor terminal fluidity, resulting in the protruding strips 133 being fragmentary or incomplete, the aforementioned feed channel 16 is extended to the periphery of the air inlet 11, as shown in FIG. 7, and a clearance is set aside at the outer periphery of the air inlet 11. In such a way, adequate material can be supplied to the interior of the mold to form the anti-slip portions 13a, 13b and an overlaid section 17 on the periphery of the air inlet 11. The burr formed from surplus material can be removed after cooling, and the overlaid section 17 can also serve as a second non-slip portion.

The medical use face mask of the present invention is provided with the aforementioned anti-slip portions which are connected to the soft mouth-shaped piece to form an integral body, thereby facilitating holding and use thereof by health care personnel, and preventing the face mask from easily slipping from the hands and dropping. Accordingly, for patients, the medical use face mask of the present invention provides an improvement in operational efficiency when fitting the face mask and facilitates removal when health care personnel need to examine the patient or for cleaning and sterilization, and thus advantageous in use. In addition, the anti-slip structure of the present invention can clearly also be applied to general hard facemasks.

It is of course to be understood that the embodiments described herein are merely illustrative of the principles of the invention and that a wide variety of modifications thereto may be effected by persons skilled in the art without departing from the spirit and scope of the invention as set forth in the following claims.

## Claims

1. An anti-slip face mask (1), comprising a casing (10) made from hard plastic, wherein an air inlet (11) protrudes from the top of the surface of the casing (10); and a mouth-shaped piece (12) made from soft elastic plastic formed on the opening edge portion of the casing (10); and is **characterized in that** anti-slip portions (13a, 13b) made from the same material as the mouth-shaped piece (12) and connected thereto are formed on portions of the surface of the casing.

2. The medical use face mask (1) according to claim 1, wherein the anti-slip portions (13a, 13b) and the mouth-shaped piece are formed as an integral body.

3. The medical use face mask (1) according to claim 2, wherein the anti-slip portions (13a, 13b) are provided on two side positions on the surface of the casing and positioned at the front and rear of the air inlet (11), or to the left and right of the air inlet (11).

4. The medical use face mask (1) according to claim 2, wherein the anti-slip portions (13a, 13b) are selected and structured from a plurality of thin protruding strips (131, 133), a plurality of fine protruding points (132), a plurality of sheet form bodies (134) or a mixed configuration thereof.

5. The medical use face mask (1) according to claim 2, wherein the anti-slip portions(13a, 13b) and the mouth-shaped piece (12) are formed as an integral body, and extend from portions on the surface of the casing (1) to the circumferential surface of the air inlet (11).
